# FASCICULE DE BREVET EUROPEEN

(11) **EP 1 537 147 B1**
(45) Date de publication et mention de la délivrance du brevet: **27.10.2010**
(21) Numéro de dépôt: 03773779.8
(22) Date de dépôt: 15.09.2003
(51) Int. Cl.: C07K 16/34

(54) **ANTICORPS ANTI HLA-DR AYANT UNE ADCC AUGMENTÉE ET INDUISANT LA PRODUCTION DE CYTOKINES.**
ADCC-VERMITTELNDE ANTIKÖRPER GEGEN HLA-DR, DIE DIE PRODUKTION VON ZYTOKINEN INDUZIEREN
ANTIBODY ANTI HLA-DR WITH AN INCREASED ADCC AND INDUCING CYTOKINE PRODUCTION

(30) Priorité: 13.09.2002 FR 0211415; 13.09.2002 FR 0211416; 12.06.2003 FR 0307067
(43) Date de publication de la demande: 08.06.2005
(73) Titulaire: LFB Biotechnologies, 91940 Les Ulis (FR)
(72) Inventeur: de ROMEUF, Christophe, F-59000 Lille (FR); GLACET, Arnaud, F-59147 Gondecourt (FR); LIROCHON, Jacky, F-91650 Breuillet (FR); BOUREL, Dominique, F-59110 La Madeleine (FR)
(74) Mandataire: Warcoin, Jacques
(86) Numéro de dépôt international: PCT/FR2003/002713
(87) Numéro de publication internationale: WO 2004/029092

(56) Documents cités:
- WO-A1-01/77181
- US-B1- 6 180 377
- CARTRON GUILLAUME ET AL: "Therapeutic activity of humanized anti-CD20 monoclonal antibody and polymorphism in IgG Fc receptor FcgammaRIIIa gene" BLOOD, vol. 98, no. 11 Part 1, 16 novembre 2001 (2001-11-16), page 602a, XP001193741 & 43RD ANNUAL MEETING OF THE AMERICAN SOCIETY OF HEMATOLOGY, PART 1; ORLANDO, FLORIDA, USA; DECEMBER 07-11, 2001 ISSN: 0006-4971
- VIDOVIC D ET AL: "SELECTIVE APOPTOSIS OF NEOPLASTIC CELLS BY THE HLA-DR-SPECIFIC MONOCLONAL ANTIBODY" CANCER LETTERS, NEW YORK, NY, US, vol. 128, no. 2, 19 juin 1998 (1998-06-19), pages 127-135, XP000857331 ISSN: 0304-3835
- SHIELDS R L ET AL: "Lack of fucose on human IgG1 N-linked oligosaccharide improves binding to human FcgammaRIII and antibody-dependent cellular toxicity" JOURNAL OF BIOLOGICAL CHEMISTRY, AMERICAN SOCIETY OF BIOLOGICAL CHEMISTS, BALTIMORE, MD, US, vol. 277, no. 30, 26 juillet 2002 (2002-07-26), pages 26733-26740, XP002964542 ISSN: 0021-9258
- WRIGHT A ET AL: "Effect of glycosylation on antibody function: implications for genetic engineering" TRENDS IN BIOTECHNOLOGY, ELSEVIER PUBLICATIONS, CAMBRIDGE, GB, vol. 15, no. 1, 1997, pages 26-32, XP004016809 ISSN: 0167-7799
- SHINKAWA T ET AL: "The absence of fucose but not the presence of galactose or bisecting N-acetylglucosamine of human IgG1 complex-type oligosaccharides shows the critical role of enhancing antibody-dependent cellular cytotoxicity" JOURNAL OF BIOLOGICAL CHEMISTRY, AMERICAN SOCIETY OF BIOLOGICAL CHEMISTS, BALTIMORE, MD, US, vol. 278, no. 5, 31 janvier 2003 (2003-01-31), pages 3466-3473, XP002965857 ISSN: 0021-9258

## Description

La présente description concerne des anticorps monoclonaux chimériques humanisés ou humains qui sont produits dans des lignées cellulaires sélectionnées, lesdits anticorps présentant une forte affinité pour le récepteur CD16 des cellules effectrices du système immunitaire mais également la propriété d'induire la sécrétion de cytokines et d'interleukines, qui peuvent moduler l'activité cytotoxique des cellules effectrices.

L'immunothérapie au moyen d'anticorps monoclonaux est en passe de devenir un des aspects les plus importants de la médecine. En revanche, les résultats obtenus lors d'essais cliniques apparaissent contrastés. En effet, il peut s'avérer que l'anticorps monoclonal ne soit pas suffisamment efficace. De nombreux essais cliniques sont arrêtés pour diverses causes telles que le manque d'efficacité et des effets secondaires incompatibles avec une utilisation en thérapie clinique. Ces deux aspects sont étroitement liés sachant que des anticorps peu actifs sont administrés à fortes doses pour compenser et obtenir une réponse thérapeutique. L'administration de forte dose induit non seulement des effets secondaires mais est économiquement peu viable.

Ces problèmes sont majeurs dans le développement industriel des anticorps monoclonaux, chimériques humanisés ou humains. A titre d'exemple, la société Protein Design Labs a suspendu les essais cliniques en phase I/II de l'apolizumab (Remitogen®), qui est un anticorps anti-HLA-DR pouvant être utilisé pour traiter les cancers de cellules MHC de classe II positives, notamment les leucémies des cellules B et T.

Aujourd'hui, la recherche est orientée sur le fragment Fcy de l'immunoglobuline afin d'améliorer les propriétés fonctionnelles des anticorps. A terme, cela devrait permettre l'obtention d'anticorps qui interagissent et activent les récepteurs des cellules effectrices (monocytes macrophage, lymphocyte B, cellules NK et dendritiques).

La fixation d'un anticorps sur son ligand peut induire une activation de certaines cellules effectrices via leurs récepteurs Fc, ce qui est l'objectif de la présente invention. Nous avons montré que certains anticorps non seulement supportaient des propriétés fonctionnelles, comme l'ADCC ou l'activation du complément, mais aussi induisaient la production de cytokines. Ces cytokines, produites au site d'activation des effecteurs, peuvent exercer différentes activités biologiques.

Ainsi, il apparaît nécessaire de tester les propriétés des anticorps candidats à induire la production de ces facteurs modulant la réponse immunitaire. En effet, on a trouvé que l'activation des récepteurs des cellules effectrices produit des réponses très différentes conduisant à la libération d'une ou plusieurs cytokines. Ces cytokines sont responsables de l'activation ou de l'inhibition de certains composants du système immunitaire selon le cas. Il peut s'avérer par exemple qu'un même anticorps dirigé contre un antigène donné soit complètement inefficace lors qu'il est produit dans des lignées de myélome de souris, alors qu'il se montre très efficace lors qu'il est produit dans d'autres lignées cellulaires.

Par exemple, nous démontrons ici que la fixation d'un anticorps sur son ligand peut induire une activation de la cellule Jurkat transfectée CD 16 avec comme conséquence la sécrétion d'IL2. Une forte corrélation est observée entre la sécrétion d'IL2 par Jurkat CD16 et l'activité ADCC médiée par le CD16 des cellules effectrices.

Nous avions montré dans notre demande WO 01/77181 (LFB) l'importance de sélectionner des lignées cellulaires permettant de produire des anticorps présentant une forte activité ADCC du type FeγRIII (CD16). Nous avions trouvé que la modification de la glycosylation du fragment constant des anticorps conduisait à améliorer l'activité ADCC dans des lignées de myélomes de rat telle que YB2/0. Les structures glycanniques étant de type biantennées, avec des chaînes courtes, une faible sialylation, des mannoses et GlcNAc du point d'attache terminaux non intercalaires, 2a et une faible fucosylation 2b. lesdites structures glycanniques ayant une teneur supérieure à 60% pour les formes G0 + G1 + G0F +G1F : 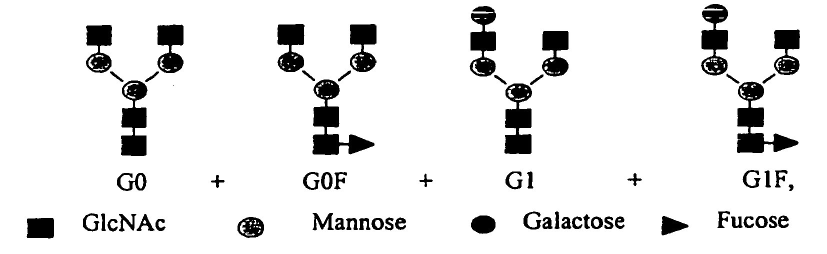 , lesdites structures glycanniques ayant une teneur inférieure à 50% pour les formes G0F +G1F

Or, dans le cadre de la présente invention, nous avons découvert que l'avantage de présenter une forte affinité pour le CD16 peut encore être renforcé par des tests supplémentaires visant à choisir les anticorps qui induisent la production de cytokines.

Les deux caractéristiques précitées se complémentent. En effet, la production de cytokines induites par les anticorps sélectionnés par le procédé de l'invention pourrait renforcer activité cytotoxique des anticorps. Le mécanisme d'action d'une telle activation tient probablement à une régulation positive autocrine des cellules effectrices. On peut postuler que les anticorps se lient au CD 16 provoquant une activité cytotoxique mais également induisent la production d'IFN gamma qui au final peut conduire à augmenter encore davantage l'activité cytotoxique.

L'invention propose donc des anticorps anti-HLA-DR qui présentent une activité jusqu'à 100 fois supérieure aux anticorps disponibles en thérapie. En particulier, l'invention apporte un anti HLA-DR significativement plus efficaces que son homologue l'apolizumab (Remitogen®) 3a. La présente invention marque un tournant majeur dans le développement des anticorps à visée clinique en apportant une nouvelle génération dont les doses efficaces pour obtenir 50% d'activité sont bien inférieures à celles des anticorps actuellement utilisés.

Ainsi, l'invention se rapporte à un anticorps anti-HLA-DR monoclonal chimérique, humanisés ou humain 3b , ledit anticorps présentant un taux ADCC de type FcγRIII (CD16) supérieur à 60 %, 70%, 80 % ou de préférence supérieur à 90 % comparé au même anticorps produit dans une lignée CHO- , **caractérisé en ce qu**'il présente une capacité à induire un taux de production Un anti-CD20 significativement plus efficace que son homologue le rituxiumab (Rituxan®) est également décrit. ayant :
- des structures glycanniques de type biantennées, avec des chaînes courtes, une faible sialylation, des mannoses et GlcNAc du point d'attache terminaux non intercalaires, lesdites structures glycanniques ayant une teneur supérieure à 60% pour les formes G0 + G1 + G0F +G1F : 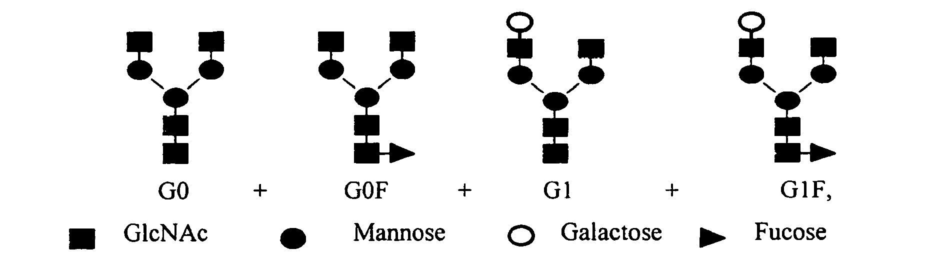 et
- une faible fucosylation, lesdites structures glycanniques ayant une teneur inférieure à 50% pour les formes G0F +G1F,

d'au moins une cytokine par une cellule effectrice du système immunitaire exprimant le récepteur CD 16 supérieur à 60 %, 100 % ou de préférence supérieur à 200 % comparé au même anticorps produit dans une lignée CHO

De préférence, cet anticorps présente un taux ADCC supérieure à 100 % à une concentration de 10ng/ml comparé au même anticorps produit dans une lignée CHO et un taux de production d'au moins une cytokine par une cellule effectrice du système immunitaire, en particulier celles exprimant le récepteur CD 16 supérieur à 1000 % à une concentration de 10ng/ml comparé au même anticorps produit dans une lignée CHO

Lesdites cytokines libérées sont des interleukines, des interférons et des facteurs de nécrose tissulaire (TNF).

Ainsi, l'anticorps est sélectionné pour sa capacité d'induire la sécrétion d'au moins une cytokine choisie parmi IL-1, IL-2, IL-3, IL-4, IL-5, IL-6, IL-7, IL-8, IL-9, IL-10,... TNFα, TGFβ, IP10 et IFNγ par les cellules effectrices du système immunitaire en particulier celles exprimant le récepteur CD 16.

De préférence, l'anticorps sélectionné a la capacité d'induire la sécrétion d' IFNγ par les cellules effectrices du système immunitaire exprimant le récepteur CD16. Le taux d' IFNγ sécrété reflète la qualité de l'anticorps fixé par le récepteur CD 16 quant à son intégrité (fonction Fc) et à sa capacité (site antigénique) de liaison à l'antigène. En outre, la sécrétion d' IFNγ contribue à renforcer l'activité cytotoxique des cellules effectrices.

Les cellules effectrices peuvent exprimer un CD 16 endogène ou être transformées. On entend par cellule transformée, une cellule modifiée génétiquement de sorte à exprimer des récepteur Fc, en particulier le récepteur CD16.

Dans un mode de réalisation particulier, l'anticorps de l'invention est capable d'induire la sécrétion d'au moins une cytokine par une cellule leucocytaire, en particulier de la famille des NK (natural killer) ou par des cellules du groupe monocytes-macrophages. De préférence, on utilise pour la sélection des anticorps une lignée Jurkat transfectée avec un vecteur d'expression codant pour le récepteur CD16 comme cellule effectrice. Cette lignée est particulièrement avantageuse car elle est immortalisée c'est à dire qu'elle se développe indéfiniment dans des milieux de cultures et qu'elle permet d'obtenir des résultats reproductibles de par sa stabilité d'expression du CD 16.

La sélection peut se faire sur des anticorps produits par des cellules couramment utilisées pour la production d'anticorps thérapeutiques, telles que les lignées de myélomes de rat, en particulier YB2/0 et ses dérivés, les cellules lymphoblastoïdes humaines, les cellules d'insectes et les cellules de myélomes murines. La sélection peut également être appliquée à l'évaluation d'anticorps produits par des plantes transgéniques ou de mammifères transgéniques. A cet effet, la production dans CHO sert de référence (CHO étant employée pour la production d'anticorps médicament) pour comparer et sélectionner les systèmes de production conduisant aux anticorps selon l'invention. Une autre alternative consiste à effectuer la comparaison avec les des anticorps dont les essais cliniques ont été arrêtés et qui se sont révélés peu efficaces et/ou produisant des effets secondaires indésirables aux doses administrées. En effet, comme indiqué précédemment, les anticorps modifiés de l'invention sont jusqu'à 100 fois plus efficaces pour activer l'ADCC des cellules effectrices du système immunitaires, ce qui implique des doses d'administration inférieures à celles pratiquées par les anticorps mentionnés précédemment.

L'anticorps de l'invention peut être produit dans des lignées cellulaires du type myélome de rat, notamment YB 2/0. Il peut être dirigé contre un antigène normal non ubiquitaire (exemple, l'anticorps est de spécificité anti- Rhésus D du globule rouge humain), ou un antigène d'une cellule pathologique ou d'un organisme pathogène pour l'homme, en particulier contre un antigène d'une cellule cancéreuse. Par L'anticorps est un anti-HLA-DR. Cet anticorps présente un taux ADCC supérieure à 100 % à une concentration de 10 ng/ml ou moins et un taux de production d'IL-2 par une cellule effectrice du système immunitaire exprimant le récepteur CD16 supérieur jusqu'à 1000 % à une concentration de 10 ng/ml comparé au même anticorps exprimé dans la lignée CHO, lignée d'expression de l'apolizumab (Remitogen®).

L'anti-HLA-DR de l'invention peut être produit dans une lignée de myélome de rat, notamment YB2/0.

Dans un autre aspect, l'invention concerne l'utilisation d'un anticorps selon l'invention tel que décrit ci-dessus, pour la fabrication d'un médicament destiné au traitement des cancers.

Plus particulièrement, l'invention vise l'utilisation d'un anticorps Anti-HLA-DR décrit plus haut pour la fabrication d'un médicament destiné au traitement des cancers des cellules MHC de classe II positives, notamment les lymphomes de cellules B, les leucémies aiguës de cellules B, le lymphome de Burkitt, le lymphome de Hodgkin, les leucémies myéloïdes, les lymphomes et leucémies de cellules T, les lymphomes non hodgkinien et les leucémies myéloïdes chroniques.

Dans un aspect préféré de l'invention, l'anticorps peut dans un premier temps être sélectionné pour son affinité au récepteur CD 16 puis testé et sélectionné tel que décrit ci-dessus pour ses propriétés à induire la production d'une cytokine, notamment l'IL-2, par les cellules Jurkat CD16 ou l'IFNγ par les cellules effectrices sanguines exprimant le CD16.

De tels anticorps possédant cette double propriété d'induire l'ADCC via le CD 16 et d'induire la production de l'IL-2 conduisent à une stimulation très importante de l'activité cytotoxique des cellules effectrices.

L'invention porte également sur l'utilisation d'un anticorps selon l'invention tel que décrit ci-dessus pour la fabrication d'un médicament destiné à induire l'expression de TNF, IFNγ, IP10, IL8 et IL-6 par les cellules effectrices naturelles du système immunitaire, ledit médicament étant utile notamment pour le traitement du cancer et des infections.

### Légendes et titres des figures :

Figure 1: Lyse ADCC des cellules Raji, induite par des anticorps anti-HLA-DR exprimés dans CHO (triangle) ou YB2/0 (carré).
Figure 2: Sécrétion d'IL2 par les cellules Jurkat CD16, induite par des anticorps anti-HLA-DR exprimés dans CHO (triangle) ou YB2/0 (carré).
Figure 3: Corrélation entre le pourcentage d'ADCC supportée par les cellules NK et la sécrétion d'IL2 par les cellules Jurkat CD16.
Figure 4: IL8 sécrétée par les CMN en présence ou absence de cible
Figure 5: Sécrétion de cytokines par les CMN induite par les anticorps anti-Rhésus (valeur sans cible déduite) Tox 324 03 062
Figure 6: Sécrétion de cytokines par les polynucléaires induite par les anticorps anti-Rhésus
Figure 7: Sécrétion de cytokines par les NK induite par les anticorps anti-Rhésus
Figure 8: Sécrétion de TNF alpha par les cellules NK, induite par les anticorps anti-CD20 et anti-HLA-DR exprimés dans CH et YB2/0 (324 03 082)
Figure 9: Sécrétion d'IFN gamma par les cellules NK, induite par les anticorps anti-CD20 et anti-HLA-DR exprimés dans CH et YB2/0 (324 03 082)

### Exemple 1 : Anti-HLA-DR

### 1.1 TEST ADCC CD16

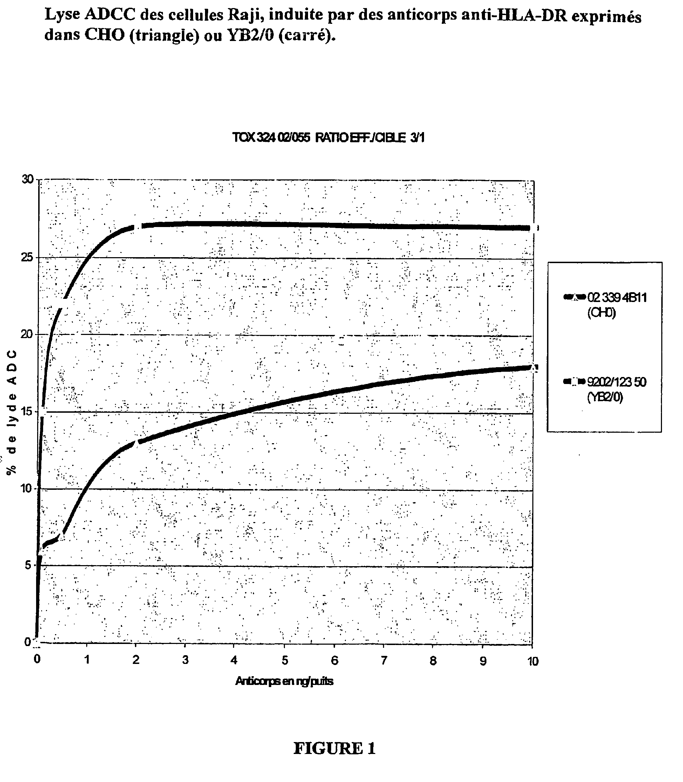

L'anticorps anti-HLA DR chimérique a été exprimé dans la cellule YB2/0 et dans CHO. Les anticorps chimériques anti-HLA-DR sont capables d'induire une activité cytotoxique de la cellule RAJI, exprimant à sa surface des antigènes HLA-DR. Pour ce faire, la même séquence codant pour une IgG1 spécifique de l'antigène HLA-DR est transfectée dans CHO et YB2/0. Les anticorps sont incubés avec les cellules RAJI (cibles) et les cellules Natural Killer (NK) humaines. L'activité cytotoxique des anticorps sur la cellule Raji (ADCC) a été évaluée après 16h d'incubation (voir figure 1).

Les deux anticorps anti-HLA-DR exprimés par YB2/0 (carré) ou CHO (triangle) induisent une lyse de la cellule Raji par ADCC. L'anticorps exprimé dans YB2/0 s'avère plus cytotoxique que CHO surtout dans des conditions de faibles quantités d'effecteurs et de faibles concentrations d'anticorps.

### 1.2 TEST IL-2

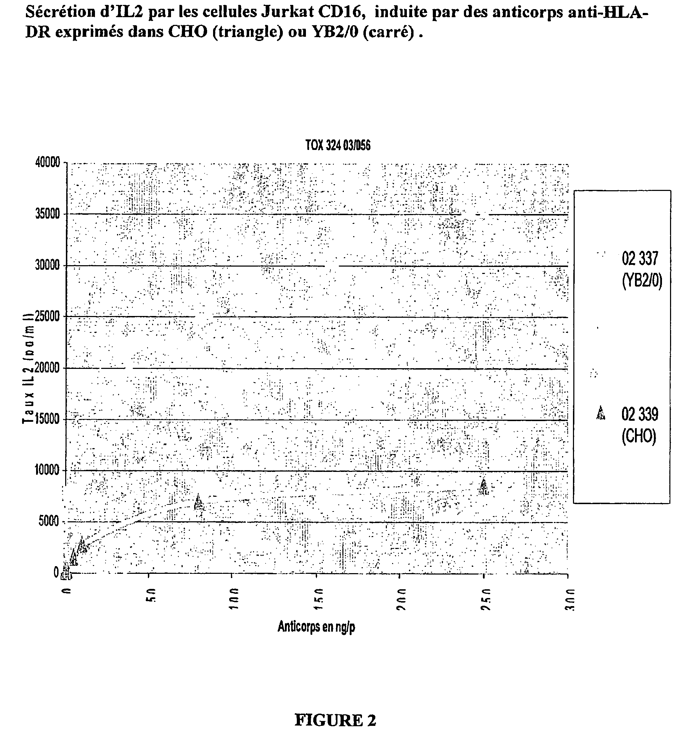

La même séquence codant pour une IgG1 spécifique de l'antigène HLA-DR est transfectée dans CHO et YB2/0. Les anticorps sont incubés avec les cellules RAJI (cible) et les cellules Jurkat CD16 (effecteurs) portant l'acide aminé phénylalanine (F) en position 158. La quantité de cytokines (IL2) sécrétée par Jurkat CD16 est mesurée par ELISA (voir figure 2).

Les anticorps anti-HLA DR induisent une forte sécrétion d'IL2 (cytokine). Comparativement, la sécrétion et donc le degré d'activation est supérieur lorsque l'anticorps est exprimé dans YB2/0 (carré) par rapport à CHO (triangle) à toutes les concentrations étudiées.

### Exemple 2 : Corrélation in vitro entre ADCC et libération d'IL-2 de Jurkat CD16.

Pour cette étude, 3 anticorps monoclonaux (Mab) anti-D ont été comparés. Le Mab DF5-EBV a été produit par des Lymphocytes B humains obtenus chez un donneur immunisé D-négatif et immortalisés par transformation avec l'EBV. Cet anticoprs a été utilisé comme contrôle négatif étant donné que lors d'un essai clinique, il a été montré incapable d'éliminer les globules rouges rhésus positifs de la circulation.

L'anticorps monoclonal (Mab) DF5-YB2/0 a été obtenu en exprimant la séquence primaire de DF5-EBV dans la lignée YB2/0. L'anticorps monoclonal R297 et d'autres anticorps recombinants ont également été exprimés dans YB2/0.

Ces anticorps sont testés in vitro pour leur capacité à induire une lyse des globules rouges traités à la papaïne en utilisant des cellules mononucléées (PBL) comme effecteur.

Tous les tests ont été effectués en présence d'immunoglobulines humaines (IVIg) de sorte à reconstituer les conditions physiologiques.

On pense que les IVIg se lient avec une haute affinité au FcgammaRI (CD64). Les deux Mab DF5-YB2/0 et R297 induisent une lyse des globules rouges à un niveau comparable à celui des anticorps polyclonaux WinRho. En revanche, le Mab DF5-EBV est complètement inefficace.

Dans une deuxième série d'expérience, des cellules NK purifiées et des globules rouges non traités ont été utilisés comme effecteur et cibles respectivement. Après 5 heures d'incubation, les Mabs antiD-R297 et DF5-YB2/0 se sont montrés capables de provoquer la lyse des globules rouges, alors que DF5-EBV reste inefficace.

Dans ces deux expériences, la lyse des globules rouges a été inhibée par le Mab 3G8 dirigé contre le FcgammaRIII (CD 16).

En résumé, ces résultats démontrent que l'ADCC provoquée par Mab R297 et Mab DF5-YB2/0 implique le FcgammaRIII exprimé à la surface des cellules NK.

Dans le cadre de l'invention, une troisième série d'expériences a été réalisé selon un test in vitro à l'aide de cellule Jurkat CD16 pour évaluer l'efficacité d'anticorps anti-D. Les Mab ont été incubés pendant une nuit avec des globules rouges Rhésus positif et des cellules Jurkat CD16. La libération d'IL-2 dans le surnageant à été évaluée par ELISA.

Une forte corrélation entre l'ADCC et l'activation des cellules Jurkat a été observée, ce qui implique que ce test peut être utilisé pour faire la discrimination des Mabs anti-D en fonction de leur réactivité envers FcgammaRIII ( CD16).

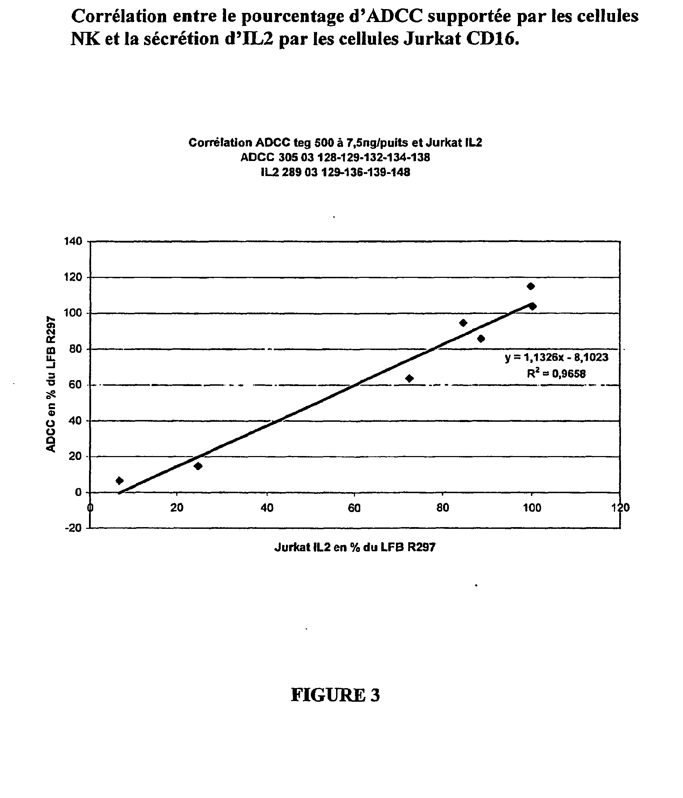

Les mêmes échantillons sont évalués en ADCC et dans le test Jurkat IL2. Les résultats sont exprimés en pourcentage de l'anticorps de référence "LFB-R297". La courbe de corrélation entre les 2 techniques a un coefficient r2=0.9658 (Figure 3).

En conclusion, ces données montrent l'importance des modifications post-traductionnelles de la partie Fc des anticorps pour leur capacité à induire une activité ADCC spécifique du FcgammaRIII. La libération de cytokines telles que l'IL-2 est corrélée avec l'ADCC.

### Exemple 3 : activation de cellules NK et production d'IL2 et d'IFNγ

Modèle de mise au point : lignée cellulaire Jurkat transfectée avec le gène codant pour le récepteur CD16. Applications : renforcement d'une réponse anti-tumorale. L'IL2 produite par les cellules effectrices activées induit une activation des lymphocytes T et des cellules NK pouvant aller jusqu'à une stimulation de la prolifération cellulaire. L'IFNγ stimule l'acdvité des CTLs et peut renforcer l'activité des macrophages.

### Exemple 4 : activation de monocytes macrophages et production de TNF et d'IL-1Ra

Applications : renforcement de la phagocytose et induction de propriétés anti-inflammatoires. Le TNF produit par les cellules effectrices activées stimule la prolifération des macrophages et des lymphocytes infiltrant les tumeurs. L'IL-1Ra est une cytokine qui entre en compétition avec l'IL1 au niveau de son récepteur et exerce ainsi un effet anti-inflammatoire.

### Exemple 5 : activation de cellules dendritiques et production d'IL10

Applications : induction d'une tolérance spécifique à certains antigènes. L'IL10 est une molécule inhibitrice de l'activation de différentes cellules effectrices et de la production de cytokines.

### Exemple 6 : Induction de la sécrétion de cytokines par différentes cellules effectrices.

Trois populations cellulaires ont été étudiées : **les polynucléaires, les cellules mononuclées et les cellules NK**. L'induction de la synthèse de cytokines est dépendante de la présence de la cible. Il y a peu de différences dans les profils du R297 et de l'anticorps polyclonal anti-Rhésus D, en terme de cytokine sécrétées par les cellules effectrices. AD1 est très souvent non inducteur de sécrétion de cytokines.

### Résultats :

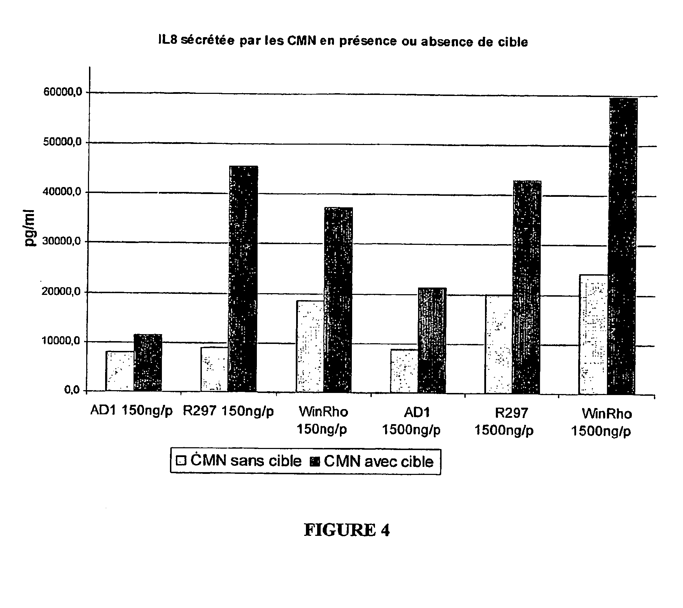

6.1 L'anticorps monoclonal anti-Rh D R297 et le polyclonal anti-Rh D WinRho induisent une sécrétion importante d'IL8 en présence de cellules mononucléées. Cette sécrétion est dépendante de la concentration d'anticorps et de la présence de la cible antigénique. L'anticorps AD1 est beaucoup moins efficace à induire la production d'IL8 par les cellules mononucléées (figure 4).

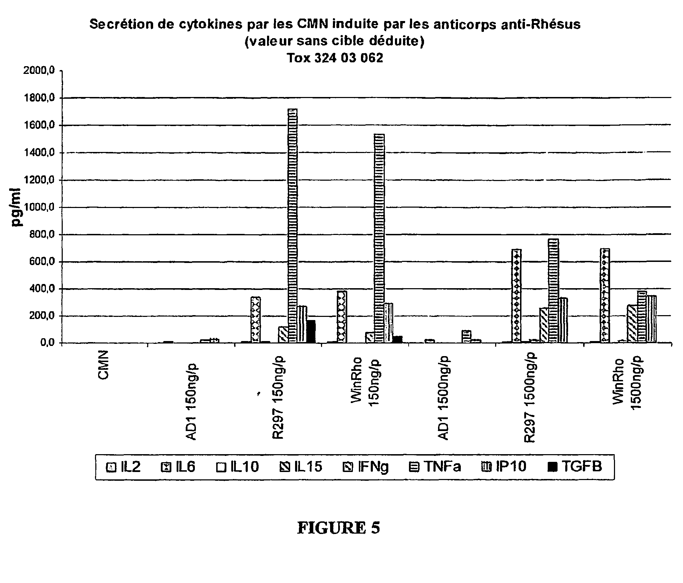

L'anticorps monoclonal anti-Rh D R297 et le polyclonal anti-Rh D WinRho induisent une sécrétion importante de TNF alpha, une sécrétion d'IL6, IFN gamma, IP10, TNF alpha et TGF Beta moins forte bien que supérieure à celles induites par AD1 par les cellules mononucléées. Cette sécrétion augmente avec la plus forte concentration d'anticorps pour l'IL6, IFN gamma, IP10, mais décroît pour le TNF alpha et le TGF Beta (figure 5).

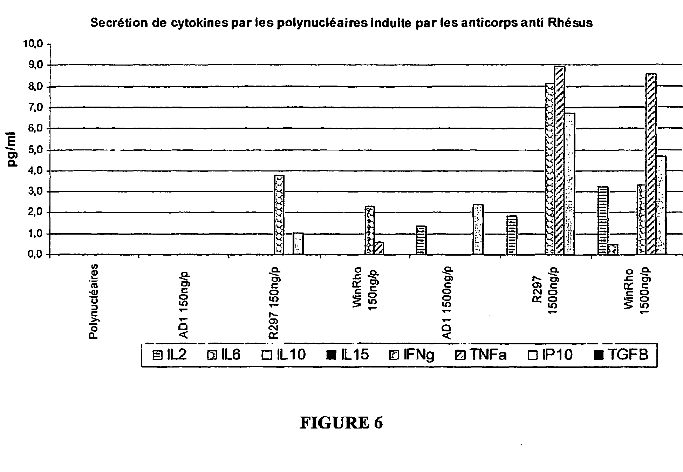

6.2 L'anticorps monoclonal anti-Rh D R297 et le polyclonal anti-Rh D WinRho induisent une sécrétion très faible, mais supérieure à AD1, d'IL2, IFN gamma, IP10 et TNF par les polynucléaires. Cette sécrétion est dépendante de la concentration d'anticorps (figure 6).

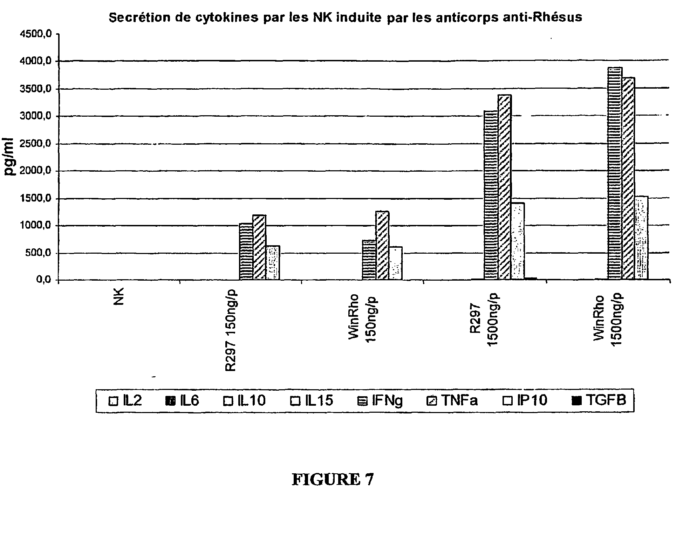

6.3 L'anticorps monoclonal R297 et le polyclonal WinRho induisent une sécrétion importante d'IFN gamma, IP10 et TNF par les cellules NK. Cette sécrétion est dépendante de la concentration d'anticorps (figure 7).

### Exemple 7 : Anticorps anti-CD 20 et anti-HLA DR chimériques optimisés produits dans YB2/0

### Introduction

Nos premiers résultats ont montré que les anticorps anti-D produits dans YB2/0 ainsi que les anticorps polyclonaux utilisés en clinique induisaient une forte activité ADCC ainsi que la production de cytokines, en particulier de TNF alpha et d'interféron gamma (IFN gamma) à partir de cellules NK purifiées ou de cellules mononuclées. Par contre d'autres anticorps anti-D, produits dans d'autres lignées cellulaires sont négatifs en ADCC et se sont révélés incapables d'induire cette sécrétion de cytokines.

Les résultats complémentaires ci dessous montrent que ce mécanisme n'est pas exclusif aux anti-D en présence d'hématies Rhésus positif mais s'applique également aux anticorps anti-CD20 et anti-HLA DR exprimés dans YB2/0. L'expression dans CHO confère à l'anticorps des propriétés activatrices moins importantes. Ceci est en corrélation avec les résultats obtenus en ADCC.

### Matériel

### Anticorps.

Anti-CD20 : l'anticorps chimérique anti-CD20 transfecté dans YB2/0 est comparé à un anticorps commercial anti-CD20 produit dans CHO (Rituxan).
Anti-HLA DR : la même séquence codant pour l'anticorps chimérique anti-HLA DR est transfectée dans CHO (B11) ou YB2/0 (4B7).
*Cellules cibles* : cellules Raji exprimant à leur surface l'antigène CD20 et HLA-DR
*Cellule effectrices* : cellules NK humaines purifiées par sélection négative à partir de poches de sang humain.

### Méthode

Différentes concentrations d'anticorps anti-CD20 ou anti-HLA DR sont incubées avec les cellules Raji et les cellules NK. Après 16 heures d'incubation, les cellules sont centrifugées. Les surnageants sont dosés en TNF alpha et IFN gamma.

### Résultats :

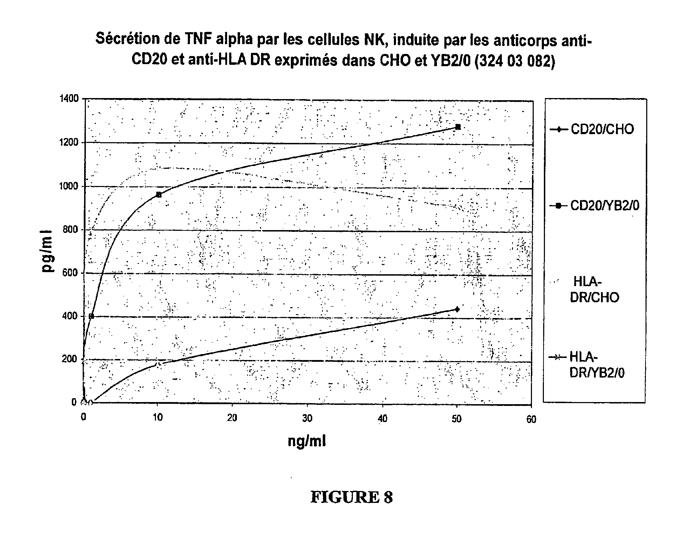

### 1) TNF alpha : Les résultats sont exprimés en pg/ml de TNF alpha dosé dans les surnageants. En abscisse figure les différentes concentrations d'anticorps ajoutées dans le mélange réactionnel (figure 8).

Les anticorps anti-CD 20 et anti-HLA DR chimériques produits dans YB2/0 induisent des taux plus importants de TNF en présence de leur cible (Raji) par rapport aux mêmes anticorps produits dans CHO. La quantité de TNF alpha est bien dose dépendante de la concentration d'anticorps ajouté. A 10ng/ml d'anticorps on induit 5 fois plus de TNF alpha avec les anticorps produits dans YB2/0 par rapport aux anticorps produits dans CHO.

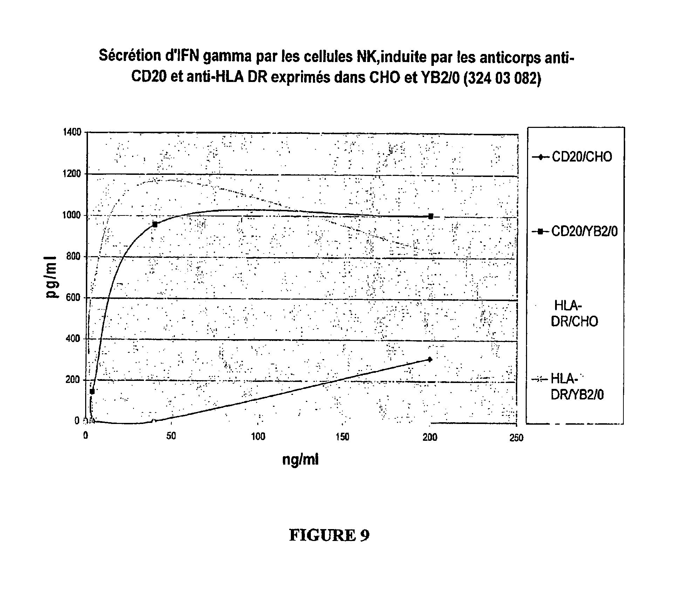

### 2) IFN gamma: Les résultats sont exprimés en pg/ml de IFN gamma dosé dans les surnageants. En abscisse figure les différentes concentrations d'anticorps ajoutées dans le mélange réactionnel (figure 9).

Les anticorps anti-CD 20 et anti-HLA DR chimériques produits dans YB2/0 induisent des taux plus importants de IFN gamma en présence de leur cible (Raji) par rapport aux mêmes anticorps produits dans CHO. La quantité de IFN gamma est bien dose dépendante de la concentration d'anticorps ajouté. A toutes les concentrations utilisées (0 à 200ng/ml) l'anticorps anti-HLA DR produit dans CHO n'induit pas de sécrétion d'IFN gamma, alors que 40ng/ml de l'anticorps produit dans YB2/0 induit environ 1000pg/ml d'IFN gamma.

Pour l'anticorps anti-CD20, il faut moins de 10ng/ml de l'anticorps produit dans YB2/0 et 200ng/ml de l'anticorps produit dans CHO pour induire 300pg/ml de IFN gamma (figure 9).

## Revendications

1. Anticorps anti-HLA-DR monoclonal chimérique, humanisé ou humain ayant :
- des structures glycanniques de type biantennées, avec des chaînes courtes, une faible sialylation, des mannoses et GlcNAc du point d'attache terminaux non intercalaires, lesdites structures glycanniques ayant une teneur supérieure à 60% pour les formes G0 + G1 + G0F +G1F : 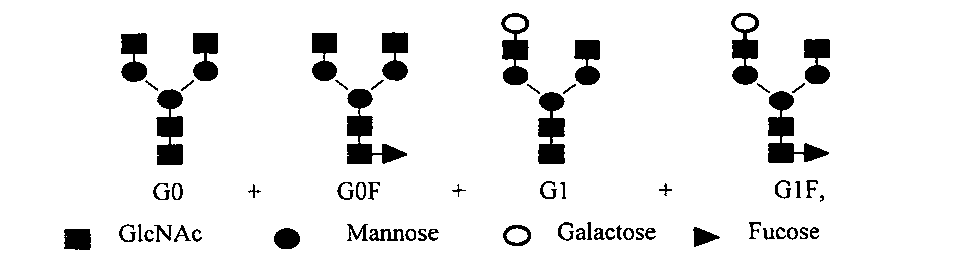 et
- une faible fucosylation, lesdites structures glycanniques ayant une teneur inférieure à 50% pour les formes G0F +G1F,
ledit anticorps présentant un taux ADCC de type FcγRIII (CD16) supérieur à 60 %, 70%, 80 % ou de préférence supérieur à 90 % comparé au même anticorps produit dans une lignée CHO, et **caractérisé en ce qu'**il présente une capacité à induire un taux de production d'au moins une cytokine par la cellule Jurkat CD16 ou une cellule effectrice du système immunitaire exprimant le récepteur CD16 supérieur à 60 %, 100 % ou de préférence supérieur à 200 % comparé au même anticorps produit dans une lignée CHO.

2. Anticorps selon la revendication 1, **caractérisé en ce qu'**il présente un taux ADCC supérieure à 100 % à une concentration 10ng/ml ou moins, comparé au même anticorps produit dans une lignée CHO et un taux de production d'au moins une cytokine par une cellule effectrice du système immunitaire exprimant le récepteur CD 16 supérieur à 1000 % à une concentration de 10ng/ml ou moins, comparé au même anticorps produit dans une lignée CHO.

3. Anticorps selon l'une des revendications 1 à 2, **caractérisé en ce que** les cytokines libérées sont des interleukines.

4. Anticorps selon l'une des revendications 1 à 2, **caractérisé en ce que** les cytokines libérées sont des interférons.

5. Anticorps selon l'une des revendications 1 à 2, **caractérisé en ce que** les cytokines libérées sont des facteurs de nécrose tissulaire (TNF).

6. Anticorps selon l'une des revendications 1 à 2, **caractérisé en ce que** l'anticorps sélectionné a la capacité d'induire la sécrétion d'au moins une cytokine choisie parmi IL-1, IL-2, IL-3, IL-4, IL-5, IL-6, IL8, TNFα , TGFβ, IP10 et IFNγ par les cellules effectrices exprimant le récepteur CD 16.

7. Anticorps selon la revendication 1 ou 2, **caractérisé en ce que** ce que l'anticorps sélectionné a la capacité d'induire la sécrétion d'IL-2 par les cellules effectrices du système immunitaire exprimant le récepteur CD 16.

8. Anticorps selon la revendication 1, 2 ou 7, **caractérisé en ce que** la cellule effectrice est une cellule Jurkat exprimant le récepteur CD 16 ou une cellule leucocytaire, en particulier de la famille des NK (natural killer) ou une cellules du groupe monocytes-macrophages.

9. Anticorps selon l'une des revendications 1 à 8, **caractérisé en ce qu'**il présente un taux ADCC supérieur à 100 % à une concentration de 10 ng/ml ou moins et un taux de production d'IL-2 par une cellule effectrice du système immunitaire exprimant le récepteur CD16 supérieur jusqu'à 1000 % à une concentration de 10 ng/ml ou moins comparé au même anticorps exprimé dans la lignée CHO.

10. Anticorps selon l'une des revendications 1 à 9, **caractérisé en ce qu'**il est produit dans une lignée cellulaire du type myélome de rat.

11. Anticorps selon la revendication 10, **caractérisé en ce que** ladite lignée cellulaire du type myélome de rat est YB2/0.

12. Utilisation d'un anticorps selon l'une des revendications 1 à 11 pour la fabrication d'un médicament destiné au traitement des cancers.

13. Utilisation d'un anticorps selon l'une des revendications 1 à 11 pour la fabrication d'un médicament destiné au traitement des cancers des cellules MHC de classe II positives, notamment les lymphomes de cellules B, les leucémies aiguës de cellules B, le lymphome de Burkitt, le lymphome de Hodgkin, les leucémies myéloïdes, les lymphomes et leucémies de cellules T, les lymphomes non hodgkinien et les leucémies myéloïdes chroniques.

14. Utilisation d'un anticorps selon l'une des revendications 1 à 11 pour la fabrication d'un médicament destiné à induire l'expression de TNF, IFNγ, IP10 et IL-6 par les cellules effectrices naturelles du système immunitaire, ledit médicament étant utile notamment pour le traitement du cancer.

## Claims

1. Human, humanized, or chimeric monoclonal anti-HLA-DR antibody having:
- glycan structures of the bi-antennary type, with short chains, a low degree of sialylation, and nonintercalated terminal attachment point mannoses and GlcNAcs, said glycan structures having a content of greater than 60% for G0 + G1 + G0F + G1F forms: 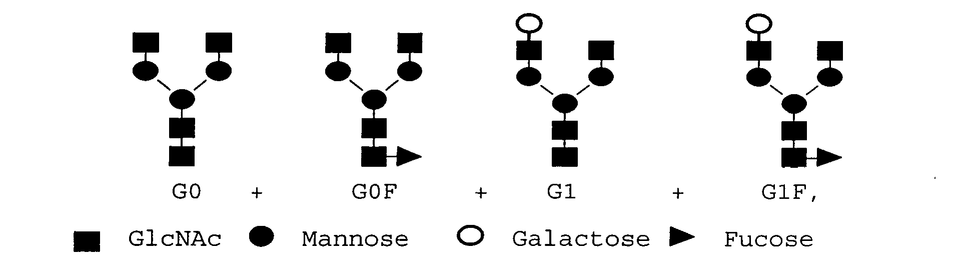 and
- a low degree of fucosylation, said glycan structures having a content of less than 50% for G0F + G1F forms,
said antibody having an FcγRIII (CD16)-type ADCC rate of greater than 60%, 70%, 80% or preferably greater than 90%, compared with the same antibody produced in a CHO line, **characterized in that** it has an ability to induce a rate of production of at least one cytokine by Jurkat CD16 cell or by a CD16 receptor-expressing effector cell of the immune system of greater than 60%, 100%, or preferably greater than 200%, compared with the same antibody produced in a CHO line.

2. Antibody according to Claim 1, **characterized in that** it has an ADCC rate of greater than 100% at a concentration of 10 ng/ml or less, compared with the same antibody produced in a CHO line, and a rate of production of at least one cytokine by a CD16 receptor-expressing effector cell of the immune system of greater than 1000% at a concentration of 10 ng/ml or less, compared with the same antibody produced in a CHO line.

3. Antibody according to either of Claims 1 and 2, **characterized in that** the cytokines that are released are interleukins.

4. Antibody according to either of Claims 1 and 2, **characterized in that** cytokines that are released are interferons.

5. Antibody according to either of Claims 1 and 2, **characterized in that** the cytokines that are released are tissue necrosis factors (TNFs).

6. Antibody according to either of Claims 1 and 2, **characterized in that** the selected antibody selected has the ability to induce the secretion of at least one cytokine chosen from IL-1, IL-2, IL-3, IL-4, IL-5, IL-6, IL-8, TNFa, TGFβ, IP10 and IFNγ, by the CD16 receptor-expressing effector cells.

7. Antibody according to Claim 1 or 2, **characterized in that** the selected antibody has the ability to induce the secretion of IL-2 by CD16 receptor-expressing effector cells of the immune system.

8. Antibody according to Claim 1, 2 or 7, **characterized in that** the effector cell is a CD16 receptor-expressing Jurkat cell or a leukocytic cell, in particular of the NK (natural killer) family, or a cell of the monocyte-macrophage group.

9. Antibody according to one of Claims 1 to 8, **characterized in that** it has an ADCC rate of greater than 100% at a concentration of 10 ng/ml or less, and a rate of IL-2 production by a CD16-receptor-expressing effector cell of the immune system of greater than up to 1000% at a concentration of 10 ng/ml or less, compared with the same antibody expressed in the CHO line.

10. Antibody according to one of Claims 1 to 9, **characterized in that** it is produced in a cell line of the rat myeloma type.

11. Antibody according to Claim 10, **characterized in that** said cell line of the rat myeloma type is YB2/0.

12. Use of an antibody according to one of Claims 1 to 11, for producing a medicinal product intended for the treatment of cancers.

13. Use of an antibody according to one of Claims 1 to 11, for producing a medicinal product intended for the treatment of cancers of MHC class II-positive cells, in particular B-cell lymphomas, acute B-cell leukemias, Burkitt's lymphoma, Hodgkin's lymphoma, myeloid leukemias, T-cell lymphomas and leukemias, non-Hodgkin's lymphomas, and chronic myeloid leukemias.

14. Use of an antibody according to one of Claims 1 to 11, for producing a medicinal product intended to induce the expression of TNF, IFNγ, IP10 and IL-6 by natural effector cells of the immune system, said medicinal product being useful in particular for the treatment of cancer.

## Patentansprüche

1. Monoclonaler chimärischer, humanisierter oder humaner Anti-HLA-DR-Antikörper, umfassend:
- Glycanstrukturen des Typs mit zwei Antennen mit kurzen Ketten, einer schwachen Sialylierung, nicht interkalären terminalen Mannosen und GlcNAc des Befestigungspunkts, wobei die Glykanstrukturen einen Gehalt von mehr als 60% für die Formen G0 + G1 + G0F + GIF aufweisen: 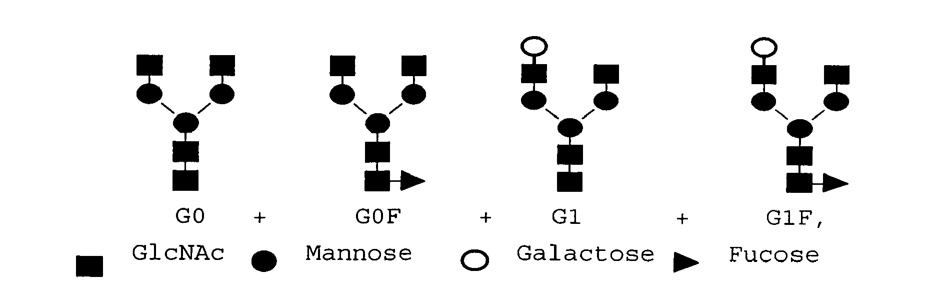 und
- eine schwache Fucosylierung, wobei die Glykanstrukturen einen Gehalt von weniger als 50% für die Formen G0F + GIF aufweisen,
wobei der Antikörper eine ADCC-Rate des Typs FcγRIII (CD 16) aufweist, die größer als 60%, 70%, 80% oder vorzugsweise größer als 90%, verglichen mit dem gleichen Antikörper, produziert in einem CHO-Stamm, ist, und **dadurch gekennzeichnet, dass** er die Fähigkeit aufweist, eine Produktionsrate von mindestens einem Zytokin durch die Jurkat CD16-Zelle oder eine Effektorzelle des Immunsystems, die den Rezeptor CD16 ausdrückt, zu induzieren, die größer als 60%, 100% oder vorzugsweise größer als 200% verglichen mit dem gleichen Antikörper, produziert in einem CHO-Stamm, ist.

2. Antikörper nach Anspruch 1, **dadurch gekennzeichnet, dass** er eine ADCC-Rate aufweist, die größer als 100% bei einer Konzentration von 10ng/ml oder weniger ist, verglichen mit dem gleichen Antikörper, produziert in einem CHO-Stamm, und eine Produktionsrate von mindestens einem Zytokin durch eine Effektorzelle des Immunsystems, die den Rezeptor CD16 ausdrückt, die größer als 1000% mit einer Konzentration von 10ng/ml oder weniger ist, verglichen mit dem gleichen Antikörper, produziert in einem CHO-Stamm.

3. Antikörper nach einem der Ansprüche 1 bis 2, **dadurch gekennzeichnet, dass** die freigesetzten Zytokine Interleukine sind.

4. Antikörper nach einem der Ansprüche 1 bis 2, **dadurch gekennzeichnet, dass** die freigesetzten Zytokine Interferone sind.

5. Antikörper nach einem der Ansprüche 1 bis 2, **dadurch gekennzeichnet, dass** die freigesetzten Zytokine Gewebenekrosefaktoren (TNF) sind.

6. Antikörper nach einem der Ansprüche 1 bis 2, **dadurch gekennzeichnet, dass** der gewählte Antikörper die Fähigkeit hat, die Sekretion von mindestens einem Zytokin, gewählt aus IL-1, IL-2, IL-3, IL-4, IL-5, IL-6, IL8, TNFα, TGFβ, IP10 und IFNγ durch die Effektorzellen, die den Rezeptor CD16 ausdrücken, zu induzieren.

7. Antikörper nach Anspruch 1 oder 2, **dadurch gekennzeichnet, dass** der gewählte Antikörper die Fähigkeit hat, die Sekretion von IL-2 durch die Effektorzellen des Immunsystems, die den Rezeptor CD16 ausdrücken, zu induzieren.

8. Antikörper nach Anspruch 1, 2 oder 7, **dadurch gekennzeichnet, dass** die Effektorzelle eine Jurkat-Zelle ist, die den Rezeptor CD16 ausdrückt, oder eine Leukozytenzelle, insbesondere der Familie der NK (natural killer) oder eine Zelle der Gruppe Monozyten-Makrophagen.

9. Antikörper nach einem der Ansprüche 1 bis 8, **dadurch gekennzeichnet, dass** er eine ADCC-Rate aufweist, die größer als 100% bei einer Konzentration von 10 ng/ml oder weniger ist, sowie eine Produktionsrate von IL-2 durch eine Effektorzelle des Immunsystems, die den Rezeptor CD16 ausdrückt, die größer als 1000% mit einer Konzentration von 10 ng/ml oder weniger ist, verglichen mit dem gleichen Antikörper, ausgedrückt im CHO-Stamm.

10. Antikörper nach einem der Ansprüche 1 bis 9, **dadurch gekennzeichnet, dass** er in einem Zellstamm des Typs Rattenmyelom produziert wird.

11. Antikörper nach Anspruch 10, **dadurch gekennzeichnet, dass** der Zellstamm des Typs Rattenmyelom YB2/0 ist.

12. Verwendung eines Antikörpers nach einem der Ansprüche 1 bis 11 für die Herstellung eines Arzneimittels, das für die Behandlung von Krebserkrankungen bestimmt ist.

13. Verwendung eines Antikörpers nach einem der Ansprüche 1 bis 11 für die Herstellung eines Arzneimittels, das für die Behandlung der Krebserkrankungen der positiven MHC-Zellen Klasse II bestimmt ist, insbesondere der B-Zellen-Lymphome, der akuten B-Zellen-Leukämien, des Burkitt-Lymphoms, des Hodgkin-Lymphoms, der myeloischen Leukämien der T-Zellen-Lymphome und Leukämien, der nicht Hodgkin'schen Lymphome und der chronischen myeloischen Leukämien.

14. Verwendung eines Antikörpers nach einem der Ansprüche 1 bis 11 für die Herstellung eines Arzneimittels, das dazu bestimmt ist, die Expression von TNF, IFNγ, IP10 und IL-6 von den natürlichen Effektorzellen des Immunsystems zu induzieren, wobei das Arzneimittel insbesondere für die Behandlung von Krebs geeignet ist.
